# EUROPEAN PATENT APPLICATION

(11) **EP 2 626 042 A1**
(43) Date of publication of application: **14.08.2013**
(21) Application number: 13150112.4
(22) Date of filing: 03.01.2013
(51) Int. Cl.: A61F 2/36, A61F 2/30

(54) **Femoral prosthesis with an offset head**

(30) Priority: 10.02.2012 GB 201202367
(71) Applicant: Stryker Ireland Limited, Carrigtwohill, County Cork (IE)
(72) Inventor: Cueille, Christophe Jean Francois, 14210 Missy, Caen (FR)
(74) Representative: Bridge-Butler, Jeremy

(57) **Abstract**

A femoral prosthesis comprising a stem part and a head part, in which the stem part comprises a body section for location in the femur of a patient and a neck section comprising a spigot, in which the head part comprises a bearing surface formed as part of a sphere and a reverse side, in which the bearing surface comprises a polar axis extending through a centre of the sphere formed thereby, in which a bore is provided in said reverse side adapted to receive said spigot, in which said bore comprise a bore axis, and in which said bore axis is oblique to said polar axis.

## Description

The present invention relates to a femoral prosthesis for use in hip joint replacement procedures.

Femoral prostheses comprise a stem part and a head part. The stem part comprises a body section for location in the intramedullary cavity of the patient's femur, and a neck section at its upper end for carrying the head part. The head part comprises a bearing surface formed as part of a sphere, which is for articulation in a cup component implanted into the patient's acetabulum. The stem part and the head part are separate components which are connected together during the procedure. The neck section of the stem part comprises a tapered spigot, and a reverse side of the head part comprises a tapered bore adapted to receive said spigot. This configuration allows for the femoral stem to be more readily implanted into the femur initially, and for a particular head part to then be chosen to suit during the procedure.

The body section of the stem part comprises a body axis which is generally aligned with an axis of the patient's femur, and the spigot is provided on the neck section at neck axis which is at an offset angle from said body axis. This offset angle must be such that the bearing surface is correctly aligned with the cup component. Misalignment can lead to problems such as excessive wear or local tissue damage post procedure, and revision surgery may then be required. The degree of the offset angle also determines the resulting leg length and the soft tissue tension across the joint, and again if this is not right then it can lead to problems post-procedure. Therefore, a femoral prosthesis with the correct offset angle is selected to suit the requirements of the patient. Further, during the procedure one of a plurality of stem parts and head parts of a modular set can be selected to reach the required result.

However, all patents are different and traditional stem and head configurations of this kind only provide a limited number of possibilities. In order to provide greater flexibility it is known for femoral prostheses to comprise a stem part, a plurality of selectable intermediate neck parts and a head part. The stem part comprises a tapered spigot, and a reverse side of each intermediate neck part comprises a tapered bore adapted to receive said spigot. Therefore, the connection between the stem part and each particular intermediate neck part is the same each time. However, the intermediate neck parts each comprise a spigot for carrying the head part which is arranged at a different offset angle, and/or at a different position, to the body axis of the stem part. Thus, the angle and/or position of the head part in relation to the body axis of the stem part can be adjusted to suit, by selecting a particular intermediate neck part. This configuration is advantageous because the stem part and the head part can manufactured to a regular shape, and the modularity can be provided by the intermediate neck parts.

However, these three-part femoral prostheses comprise two taper junctions, which significantly weakens the prosthesis and doubles the potential points of failure. There can also be metal corrosion at the taper junctions leading to Co and Cr ions being released which can cause an inflammatory reaction in the patient. Further, there are only a certain number of possibilities which can practically be provided in a set, and micro adjustment between the fixed parts is not possible.

It is also known to introduce modularity into the head parts of femoral prostheses. Such an arrangement is shown in US 2010/0152860 in the name of Brooks et al, in which the tapered bore of the head part is displaced from the polar axis of the bearing surface thereof, in a plane which is normal to said polar axis. The distance from i) the point on the tapered surface of the spigot at or towards its free end at which the tapered surface thereof no longer contacts the tapered surface of the bore, to ii) the centre of the sphere which is defined by the bearing surface, measured from said point on the spigot parallel to the axis of the spigot towards the intramedullary cavity, when the spigot is received in the bore of the head part, is at least 5mm, with said point on the tapered surface of the spigot being located between the centre of the sphere and the bearing surface of the head part. This configuration allows for the position of the head part in relation to the stem part to be set by choosing a particular rotational relationship therebetween. To move the head part closer the stem part the bore can be lowermost, and to move the head part further away it can be uppermost.

However, there are three problems with the device shown in Brooks. Firstly, it does not provide any angular adjustment between the head part and the stem part. The position of the head part can only be adjusted within the plane of the free end of the spigot. Secondly, by displacing the axis of the bore from the polar axis of the bearing surface, any rotational loading of the head part post procedure acts to destabilise the taper joint between these parts, which is a serious extra potential cause of failure. Thirdly, the scope of adjustment provided by one free rotational facility is fairly limited, and as such in the embodiment shown in Brooks a second free rotational facility is provided by means of an intermediate spigot component for fitment between the stem part and the head part. The intermediate spigot component comprises a spigot for carrying the head part and a bore for receiving the spigot of the stem part, which are also displaced from one another. This does provide for a suitable degree of adjustment, but once again it introduces a second taper junction which suffers from the drawbacks described above.

The present invention is intended to overcome some of the above problems.

Therefore, according to a first aspect of the present invention a femoral prosthesis comprises a stem part and a head part, in which the stem part comprises a body section for location in the femur of a patient and a neck section comprising a spigot, in which the head part comprises a bearing surface formed as part of a sphere and a reverse side, in which the bearing surface comprises a polar axis extending through a centre of the sphere formed thereby, in which a bore is provided in said reverse side adapted to receive said spigot, in which said bore comprise a bore axis, and in which said bore axis is oblique to said polar axis.

Thus, the present invention provides a femoral prosthesis comprising just two parts, in which the position of the bearing surface in relation to the stem can be selected from the kinds of useful ranges previously only possible with three part femoral prostheses. This is beneficial from a manufacturing and costs point of view, but also from a performance perspective because there is only one taper junction. The implantation procedure is also simplified over that required for three part prostheses.

The feature that the bore axis is oblique to the polar axis means that the bearing surface is arranged at an angle to the stem part which is different to that achieved were these axes aligned. As such, a head part can be selected from a plurality which has the right angular characteristics to suit the patient. Further, the bore axis can be oblique to the polar axis in any direction so lateralisation, medialisation, retroversion or anteversion of the bearing surface is possible. This is achieved by fitting the head part to the spigot in a particular rotational position.

It will be appreciated that were the bore axis and the polar axis to intersect at a centre of the bearing surface, then the position of the bearing surface would effectively be no different to that achieved were these axes aligned, because the bearing surface is spherical. The reverse side would simply be offset from the polar axis first described. However, the bore axis can still intersect the polar axis at other points, and if so an angular displacement is achieved. In effect, the bearing surface is positioned at a point on a radial path about the point of intersection between the axes. As such, the adjustment is not purely angular, but also positional. If the bore axis and the polar axis intersect within the head part the extent of the adjustment would be small. However, if the bore axis and the polar axis intersect outside the head part, the extent of the adjustment is greater.

It is also possible for the bore axis to be displaced from the polar axis at all points along its length. With this configuration an additional element of positional adjustment can be achieved. This is limited to what is structurally possible as the bore cannot intersect the bearing surface, or be disposed so close that the head part is not structurally sound.

It will be appreciated that in practice a modular set of heads can be made available according to a patient's likely requirements, in some of which the bore axis can intersect the polar axis, while in others not, and the surgeon can select that which is most appropriate during the implantation procedure.

As described above, when the bore axis and the polar axis are displaced, any rotational loading of the head part post procedure may act to destabilise the taper joint between the spigot and the head part. Further, if the bearing surface needs to be positioned in a particular direction, for example laterally as opposed to medially, it is important to ensure that the head part is fitted to the spigot in the correct rotational position to achieve the desired result.

Therefore, in a preferred construction the outer surface of the spigot or an inner surface of the bore can be provided with a protrusion, the other of said outer surface or said inner surface can be provided with a co-operating indent, the protrusion can be disposed in the indent when the head part is fitted onto the spigot, and at least part of the protrusion can be radially distorted from the bore axis such that rotation of the head part about the bore axis is prevented.

The term "radially distorted" means that the protrusion comprises an irregular component in a radial direction of the bore axis which prevents rotation thereabout. It will be appreciated that this could be any shape which is not annular and aligned with the bore axis. It could comprise any asymmetrical configuration, but this would only allow for one fitting position (unless a plurality of radially dispersed indents were provided), and it would lead to an irregular loading in use. Therefore, preferably the protrusion can be symmetrical, so a plurality of possible fitting positions are provided, and so the loading is equally distributed about the interface in use. A symmetrical but radially distorted shape could be any geometric shape with sides, such as a square, but also shapes with radial extensions such as a cross or star shape.

This protrusion and indent feature ensures that the head part can be fitted at a particular pre-determined rotational position on the spigot, so the intended positional adjustment is achieved, and also that that any offset rotational loading experienced post procedure will not lead to failure of the surface contact between the spigot and the bore because these surfaces will not experience any rotational shear stress. Depending on the shape and configuration of the protrusion and the indent, this feature also allows for a particular number of specific rotational fitting positions for the head part. For example, a head part can be fitted in one of four rotational positions, each of which provides a known positional adjustment in a particular direction. As such, a head part with the correct oblique angle between the bore and polar axes can be chosen from a set, and then fitted in a particular one of its possible rotational positions to arrive at the correct formation. There is no need to estimate a correct rotational fitting position during the procedure.

The protrusion can be formed on the spigot and the indent in the bore, but in a preferred embodiment the bore can comprise a base surface at an inner end thereof, the spigot can comprise a free end face, and said protrusion can be formed on said base surface and said indent on said free end face. The formation of the protrusion and indent on these two surfaces is relatively simple to machine during manufacture.

Preferably said protrusion can comprise a central portion and one or more arm portions extending therefrom, and said indent can comprise a co-operating shape. It is not necessary for this co-operating shape to be identical, for example if the protrusion comprised two arms arranged at 180 degrees to one another, the co-operating shape could comprise a cross-shape or a shape with any number of pairs of arms arranged at 180 degrees to one another. However, to avoid structural weakness and to spread the loading in use it is preferred that the protrusion and indent are identical.

In one version of the invention the protrusion can comprise four arm portions arranged at 90 degree intervals, and the indent can comprise a co-operating shape. In another version the protrusion can comprise a geometric shape with a plurality of sides, and the indent can comprise a co-operating shape. The geometric shape can be a square or a hexagon.

Alternatively, the bore can comprise an annular and tapered bore side wall, the spigot can comprise a co-operating annular and tapered spigot side wall, and the protrusion can be disposed on said bore side wall and said indent on said spigot side wall. In effect this gives the spigot and the bore an irregular cross-sectional shape in order to prevent rotation. Although this is possible it may not be preferred because it would be harder to manufacture, and it would reduce the area of surface contact between the bore side wall and spigot side wall, weakening the taper junction. However, this configuration is possible nonetheless, and in one version the protrusion can comprise one or more walls extending from said bore side wall towards said bore axis, and the indent can comprise one or more co-operating troughs formed in the spigot side wall. There can be four walls arranged at 90 degree intervals, and there can be four co-operating troughs formed in the spigot side wall.

In an another alternative embodiment, instead of the protrusion being formed in the bore, the protrusion can be formed on said free end face of the spigot and the indent on said base surface of the bore.

However the protrusion and indent are configured, preferably the head part can comprise one or more position markings, the neck section can comprise one or more complimentary position marks, and alignment of said position markings can correspond to said protrusion being aligned for disposition with, or being disposed in, said indent. This feature improves the usability of the femoral prosthesis for the surgeon, as he can see from the outside that the head part and the spigot are correctly aligned, and there is no need to rotate the head part in situ in order to locate the correct rotational position for the protrusion to slot into the indent. Further, the markings can comprise indicia which indicate the rotational position reached, for example a number on the head part aligned with a master marking on the neck section.

It will be appreciated that in embodiments in which there is no ant-rotation protrusion and indent feature, the stem part of the femoral prosthesis can be like any known stem part. However, in embodiments in which the spigot comprises an indent or a protrusion for cooperation with a corresponding indent or protrusion respectively in the bore, the stem part is novel. Therefore, according to a second aspect of the present invention a stem part of a femoral prosthesis is provided, for use in a femoral prosthesis as claimed in any of claims 4 to 16 below.

In addition, in all instances the head part is novel, so in a third aspect of the present invention a head part of a femoral prosthesis is provided, for use in a femoral prosthesis as claimed in any of claims 1 to 16 below.

Further, it is common practice for surgeons to employ trial bearing heads which are placed on the stem part during the implantation procedure in order to ascertain the correct size and/or angle and/or position suitable for the patient in question. Once the required specification is determined, the actual head part is securely fitted to the stem part. Therefore, in a fourth aspect of the present invention a kit of parts comprises a femoral prosthesis as claimed in any of claims 1 to 16 and one or more trial heads, in which each of said one or more trial heads comprises a trial bearing surface formed as part of a sphere and a reverse side, in which the trail bearing surface comprises a polar axis extending through a centre of the sphere formed thereby, in which a bore is provided in said reverse side adapted to receive said spigot, in which said bore comprise a bore axis, and in which said bore axis is oblique to said polar axis.

The invention can be performed in various ways, but eleven embodiments will now be described by way of example and with reference to the accompanying drawings in which:
Figure 1 is a cross-sectional partial side view of a first femoral prosthesis according to the first aspect of the present invention, in a first configuration;
Figure 2 is a cross-sectional partial side view of the femoral prosthesis shown in Figure 1, in a second configuration;
Figure 3 is a cross-sectional partial side view of the femoral prosthesis shown in Figure 1, in a third configuration;
Figure 4 is a top view of the femoral prosthesis shown in Figure 1, in said third configuration;
Figure 5 is a partial front view of the femoral prosthesis shown in Figure 1, in said third configuration;
Figure 6 is a partial front view of the femoral prosthesis shown in Figure 1, in a fourth configuration;
Figure 7 is a reverse view of a first head part of a femoral prosthesis according to the third aspect of the present invention, which is used in the femoral prosthesis shown in Figure 1;
Figures 8a to 8e are reverse views of second through sixth head parts of femoral prostheses according to the third aspect of the present invention, which are for use in second through sixth femoral prostheses according to the first aspect of the present invention;
Figures 9 a and b are reverse views seventh and eighth head parts of femoral prostheses according to the third aspect of the present invention, which are for use in seventh and eighth femoral prostheses according to the first aspect of the present invention; and
Figures 10a to 10d are cross-sectional side views of the head part of the femoral prosthesis as shown in Figure 1, and ninth through eleventh head parts of femoral prostheses according to the third aspect of the present invention, which are for use with the femoral prosthesis as shown in Figure 1.

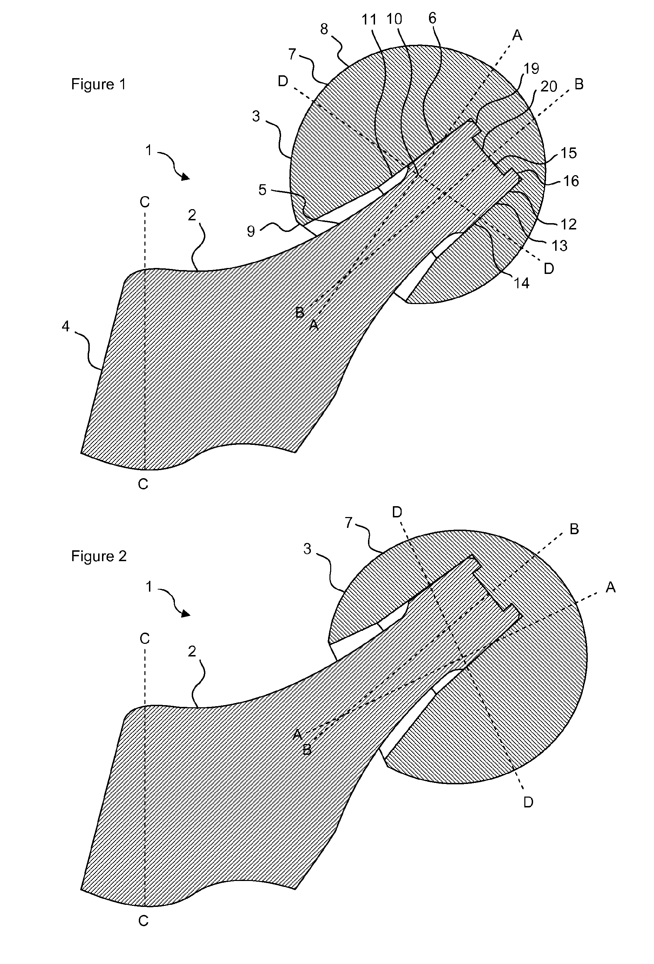

As shown in Figure 1a femoral prosthesis 1 comprises a stem part 2 and a head part 3, in which the stem part 2 comprises a body section 4 for location in the femur of a patient (not shown) and a neck section 5 comprising a spigot 6, in which the head part 3 comprises a bearing surface 7 formed as part of a sphere 8 and a reverse side 9, in which the bearing surface 7 comprises a polar axis A-A extending through a centre 10 of the sphere 8 formed thereby, in which a bore 11 is provided in said reverse side 9 adapted to receive said spigot 6, in which said bore 11 comprise a bore axis B-B, and in which said bore axis B-B is oblique to said polar axis A-A.

The stem part 2 is similar to known stem parts of femoral prostheses, and comprises a tapering body section 4 (which is only partially shown in the Figures) for location in the intramedullary cavity of the patient's femur (not shown) in the known way. The body section 4 comprises a body axis C-C, and the neck section 5 carries the spigot 6 at angle to the body axis C-C which is approximately the angle between the patient's femoral stem axis and femoral ball head polar axis. (This angle is represented in Figure 1 as the angle between axes C-C and B-B, because the bore axis B-B is aligned with spigot 6 in use.)

The head part 3 is also similar to known head parts of femoral prostheses, and comprises the part spherical bearing surface 7, which is for articulation in a cup component implanted into the patient's acetabulum (not shown) in the known way. The stem part 2 and the head part 3 are separate components which are connected together during the implantation procedure. An inner section 12 of the bore 11 and the spigot 6 comprise corresponding tapered surfaces 13 and 14 respectively. The bore 11 is introduced to the spigot 6 until the tapered surfaces 13 and 14 come into contact with one another, and an impaction force is imparted to the head part 3 to securely fix the components together, as shown in Figure 1.

The bearing surface 7 is formed as part of a sphere 8, which has a centre 10. In the Figures the line D-D represents a plane which passes through the centre 10, and which is normal to the polar axis A-A at all points thereof. As is clear from Figure 1, the bore axis B-B oblique to the polar axis A-A, and it intersects it slightly below the reverse side 9. It will be appreciated that this relationship between the bore axis B-B and the polar axis A-A applies in three dimensional space, and at all rotational positions of the head part 3 on the spigot 6, by virtue of the fact that the centre of rotation of the head part 3 is axis B-B. This is further explained below.

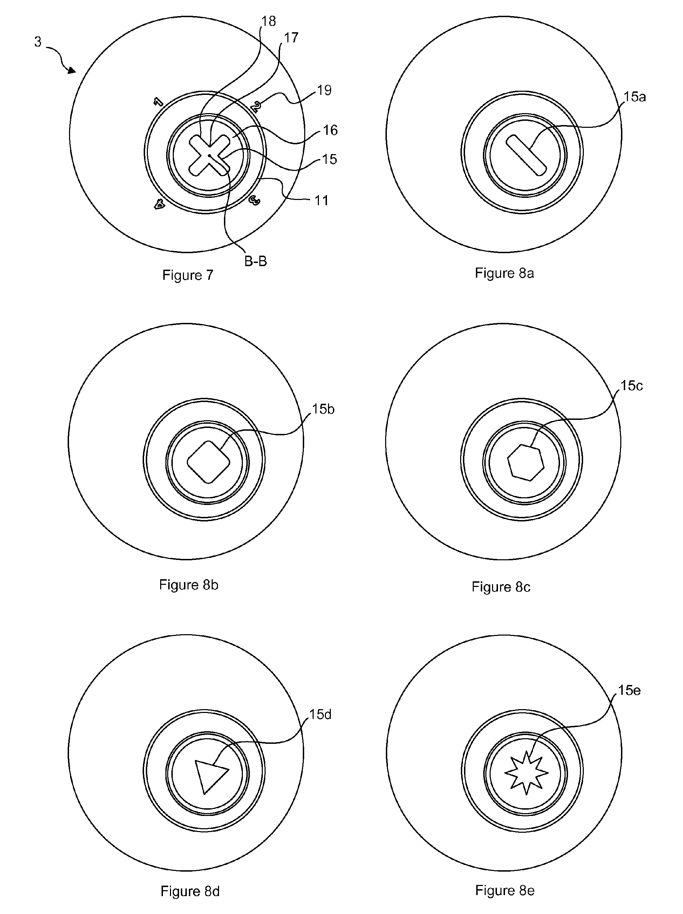

Referring to Figure 7, which shows the head part 3 from the reverse side, and therefore in line with axis B-B, a protrusion 15 is formed on a base surface 16 of the bore 11. The protrusion 15 is in the form of a cross, with a central portion 17 and four arm portions 18 extending therefrom at 90 degree intervals. This shape is radially distorted from the bore axis B-B because it does not have a regular radius about the axis B-B. This means that when this shape is interlocked with an indent of a corresponding shape, rotation therebetween is not possible. As protrusion 15 comprises two identical axes of symmetry the head part 3 can be arranged in four rotational positions on axis B-B in which the protrusion 15 assumes the same outline as shown in Figure 7. The head part 3 is provided with four position markings 19, which comprise the numerals 1-4. Each marking 19 is aligned with a particular arm portion 18 of the protrusion.

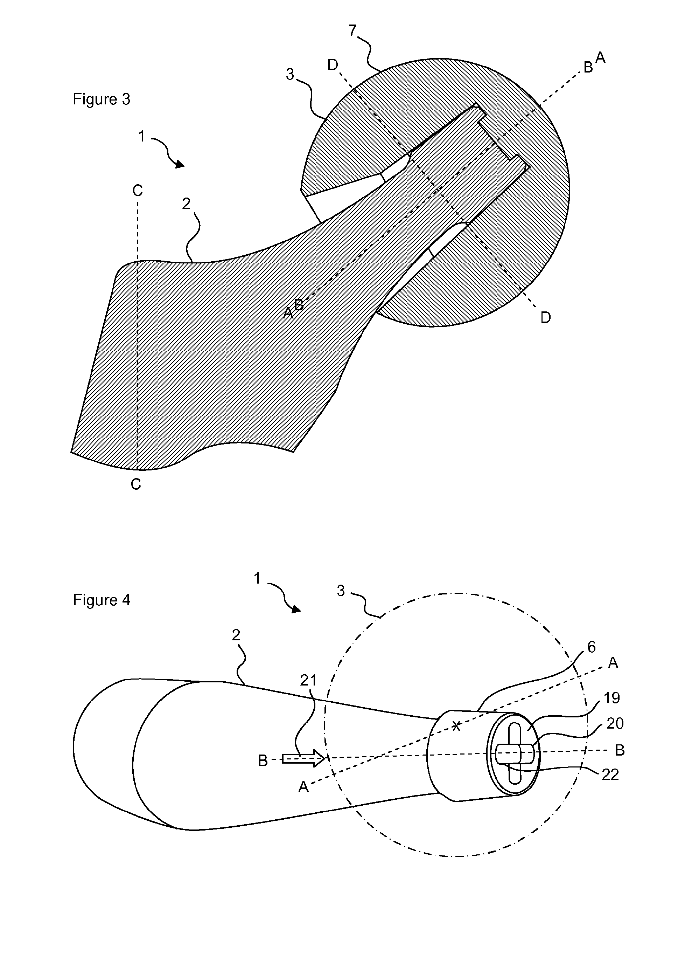

Referring to Figure 4 (which shows the head part 3 in outline only and in a different position to as shown in Figure 1) the free end face 19 of the spigot 6 comprises an indent 20. The indent 20 is of a corresponding cross shape to the protrusion 15, such that the protrusion 15 can be fitted into the indent 20. It will be appreciated that this can be done with the head part 3 in one of four possible rotational positions in relation to the spigot 4. The neck section 5 of the stem part 2 comprises a position marking 21, in the form of an arrow indicator. As is clear from Figure 4, the position marking 21 is aligned with a rearmost one of the arm portions 22 of the indent 20. Therefore, in each possible fitting position of the head part 3, the position marking 19 corresponding to the arm portion 18 which is disposed on the rearmost one of the arms 22 of the indent 20 will align with the position marking 21.

Therefore, the head part 3 can be fitted to the stem part 2 in four different ways, each of which results in the bearing surface 7 being positioned differently in relation to the bore axis B-B and the body axis C-C. In Figure 1 the head part 1 has been affixed to the spigot 6 in position 1, in which the numeral "1" position marking 19 is aligned with the arrow indicator position marking 21. In this position the bearing surface 7 is lateral to where it would be were axes B-B and A-A aligned, as in known femoral prosthesis. This position would therefore be suitable for a patient with a femoral stem axis which was relatively close to the acetabulum, or where it was required to raise the level of the bearing surface 7 slightly in relation to the fitted stem part 2 to alleviate tissue tension across the joint post procedure. It is important to note that this lateralisation involves a degree of distalisation, because the centre 10 of the sphere 8 is effectively positioned at a point on a radial path about the point of intersection between the axes B-B and A-A. As such, the bearing surface 7 is positioned both laterally and distally to where it would be were axes B-B and A-A aligned. However, this distalisation is far less than the displacement experienced in prior art head parts in which the bore axis B-B is parallel with the polar axis A-A, and the lateralisation is provided by merely displacing these axes from one another. There the lateralisation is accompanied by a significant proximalisation or distalisation. As such the femoral prosthesis 1 provides a far more useful range of lateral adjustment, which is similar to that achieved with known three part femoral prostheses.

Figure 2 shows the head part 3 fitted to the stem part 2 in position 3, in which the numeral "3" position marking 19 is aligned with the arrow indicator position marking 21. In this position the bearing surface 7 is medial to where it would be were axes B-B and A-A aligned. This position would therefore be suitable where the bearing surface 7 needed to be lower and/or further in medially than in position 1 as shown in Figure 1. Again, this medialisation involves a degree of distalisation, because the centre 10 of the sphere 8 is positioned at a point on a radial path about the point of intersection between the axes B-B and A-A. However, this is far less than in the prior art, and the medialisation is therefore more practical.

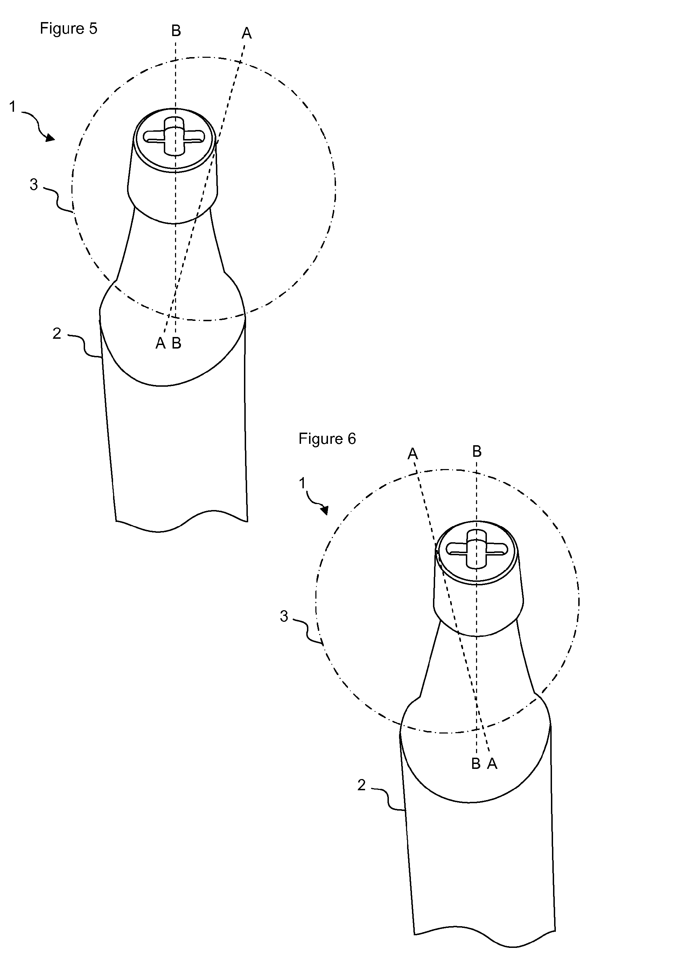

Figures 3, 4 and 5 show the head part 3 fitted to the stem part 2 in position 2, in which the numeral "2" position marking 19 is aligned with the arrow indicator position marking 21. In this position the bearing surface 7 is retroverse to where it would be were axes B-B and A-A aligned. This position would be suitable where the bearing surface 7 needed to be more rearward to suit the patient. As is clear from Figure 3, from the side view shown the axes B-B and A-A appear to overlap. However, as illustrated in the alternative views in Figures 4 and 5, which show the prosthesis 1 in perspective, it can be seen that in three dimensional space the axis A-A is oblique and offset from axis B-B. Figures 4 and 5 also illustrate the degree of retroversion achieved in position 2. As in positions 1 and 3, there is a degree of distalisation involved in the retroversion, but only to a small degree.

Figure 6 shows the head part 3 fitted to the stem part 2 in position 4, in which the numeral "4" position marking 19 is aligned with the arrow indicator position marking 21. In this position the bearing surface 7 is anteverse to where it would be were axes B-B and A-A aligned. This position would therefore be suitable where the bearing surface 7 needed to be more forward to suit the patient. As it is in perspective Figure 6 also illustrates the degree of anteversion achieved in position 4. Comparison of Figures 5 and 6 also illustrates the extent of adjustment available with head part 3. As in the other positions, there is a degree of distalisation involved in the anteversion, but only to a small degree.

Therefore, in use the surgeon can adjust the position of the bearing surface 7 between four different locations. He aligns the chosen position marker numeral 19 with the arrow indicator position marker 21, slots the head part 3 onto the spigot 6 so the protrusion 15 enters the indent 20, and then imparts an impaction force to secure the taper junction. Once in place the interface between the protrusion 15 and indent 20 prevents any possible rotation between the bore 11 and the spigot 6, which acts to prevent failure of the taper junction post procedure when rotational forces are applied which generate a shear force between the bore 11 and the spigot 6 by virtue of the misalignment of the axes B-B and A-A.

With head part 3 the difference between the four positions is relatively large, and in practice it is not likely that any given patient would require such a range of adjustment. However, Figures 1 to 6 clearly illustrate the principal of the present invention, and in particular that by making the axis B-B oblique to axis A-A, it is possible to achieve lateralisation, medialisation, retroversion and anteversion with one heat part 3. If the bore axis B-B were only oblique to the polar axis A-A, and these axes intersected within the head part, then practical micro adjustment in any of these directions would be possible.

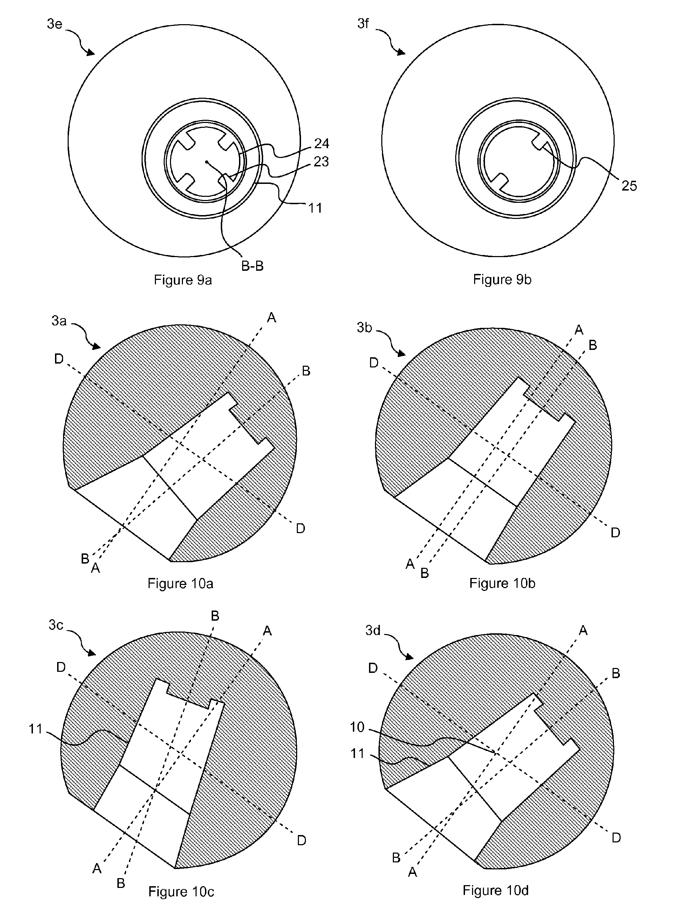

Alternatively, a set of head parts 3 may be provided, in each of which the axis B-B is oblique to axis A-A to a different extent. With such a set more appropriate adjustment in a lateral, medial, retro or ante directions can be achieved by interchanging such head parts 3 arranged in the same rotational position on the spigot 6. An illustrative set of head parts 3 is shown in Figures 10a to 10d. Head part 3a shown in Figure 10a is identical to head part 3 shown in Figures 1 to 7. However, in head part 3b the bore axis B-B is only oblique to the polar axis A-A by one degree, however there is still some space between them because they intersect some distance below the reverse side 9. In head part 3c the bore axis B-B is oblique to the polar axis A-A in the opposite direction, and the axes B-B and A-A intersect within the bore 11. In head part 3d the bore axis B-B is oblique to the polar axis A-A to the same angle as in head part 3a, except that the axes intersect higher up in the bore 11, so the extent of positional adjustment achieved is less.

With such a set of head parts 3a to 3d, a total of 16 different bearing surface 7 positions is possible, with four each in a lateral, medial, retro and ante direction to that which would be achieved if the axes B-B and A-A were aligned. If a degree of lateralisation is required then the head parts 3a to 3d can be interchanged on the spigot 6, each in position 1, to find the most suitable fit, and likewise for the other three directions.

Once again, head parts 3a to 3d may not be the most suitable for practical use, rather they are included here to illustrate the various possibilities. In practice the different arrangements between the axes B-B and A-A would be much closer together, so for example 1 mm or 2mm of adjustment was achieved between them in each particular direction. If so, the position markings on the head parts can describe the degree of positional displacement in each particular direction as part of a co-ordinated scheme used across all the head parts. For instance, instead of numerals 1-4 as shown in head part 3 in Figure 7, four head parts like those shown in Figures 10a to 10d could comprise the markings L1, L2, L3 and L4 in place of the numeral 1 respectively, to indicate a degree of lateral adjustment distance achieved, as well as the markings M1-M4, R1-R4 and A1-A4 respectively to indicate the degree of medial, retro and ante adjustment achieved in each case.

It will be appreciated also that strict lateral, medial, retro and ante adjustment may also not be appropriate. As such, in alternative embodiments (not shown) the protrusion 15 illustrated could be arranged at different rotational positions in relation to the bearing surface 7, so the head part 3 was inclined in directions between strictly lateral, medial, retro and ante.

Further, the provision of four different directional possibilities may also not be required or appropriate, and others are possible. Several alternative embodiments are illustrated in Figures 8a to 8e, and 9a and 9b. Figures 8a to 8e show head parts in which the protrusion is a different shape to a cross. In Figure 8a the protrusion 15a is a tab which passes through axis B-B, and which provides for only lateralised and medialised positions. In Figure 8b the protrusion 15b is a square, which provides for the same positions as in head part 3. In Figure 8c the protrusion 15c is a hexagon, which provides for lateralised and medialised positions as in head part 3, but also for a lateral-anteversed position, a medial-anteversed position, a lateral-retroversed position and a medial-retroversed position. In Figure 8d the protrusion 15d is a triangle which provides for a lateralised position, a lateral-anteversed position and a lateral-retroversed position. In Figure 8e the protrusion 15e is a star shape which allows for lateralised, medialised, anteversed, retroversed, lateral-anteversed, medial-anteversed, lateral-retroversed and medial-retroversed positions. In each of these cases the indents provided on the spigot of the co-operating stem parts (not shown) are corresponding shapes. It will be appreciated that in all these embodiments the protrusions 15a to 15e are radially distorted from the bore axis B-B, so rotation of the head parts on the spigots is prevented.

Figures 9a and 9b show further alternative embodiments in which the protrusion is provided in the side wall of the bore as opposed to on the base surface thereof. In head part 3e the bore 11 is provided with four walls 23 extending from a bore side wall 24 towards the bore axis B-B. The walls 23 are arranged at 90 degree intervals, and the spigot of the co-operating stem part (not shown) is provided with corresponding indents formed in its side wall. In effect, this arrangement provides the anti-rotation feature through radially distorting the cross-sectional shapes of the spigot and bore. With the four walls 23 arranged at 90 degree intervals the head part 3e can be arranged in lateralised, medialised, retroversed and anteversed positions like head part 3. Figure 9b shows a slight variation, in which only two walls 25 are provided, and as such the head part 3f can only be arranged in lateralised and medialised positions. It will be appreciated that any number of walls can be provided, and at any positions to allow for different rotational arrangements to be achieved.

The second aspect of the present invention is a stem part for use in forming a femoral prosthesis as described above. The stem part 2 shown in Figures 1 to 6 provides support for this aspect of the invention. It will be appreciated how the stem part 2 could be provided as a separate component without a head part.

The third aspect of the present invention is a head part for use in forming a femoral prosthesis as described above. The head parts 3 to 3f (as well as those shown in Figures 8a to 8e) provide support for this aspect of the invention. It will be appreciated how any of these head parts could be provided as a separate component without a stem part. It will also be appreciated how any number of these head parts can be provided as a set for a surgeon to choose from.

The fourth aspect of the present invention is a kit of parts comprising a femoral prosthesis like prosthesis 1 described above, and one or more trial heads. The trial heads comprise the same features as all the different head parts described above, although instead of an actual bearing surface they comprise a trial bearing surface. The surgeon uses the trial heads to ascertain which head part, in which rotational position, is most suitable for the patient. The required head part is then selected, and fitted in the chosen rotational position. Although the various head parts described above are actual head parts, it will be appreciated how any combination of trial heads of similar construction can be employed to provide the kit of parts described here.

The invention can be altered without departing from the scope of claim 1. For example in one alternative embodiment (not shown) the protrusion is formed on the spigot and the indent in the bore.

In another alternative embodiment (not shown) the protrusion is asymmetrical, so the head part can only be fitted to the stem part in one rotational position.

Therefore, the present invention provides a two part femoral prosthesis in which the position of the bearing surface can be usefully adjusted as in known three part prostheses. This is beneficial from a manufacturing and costs point of view, but also from a performance perspective because there is only one taper junction. The implantation procedure is also simplified over that required for three part prostheses. Further, unlike with known three-part prostheses, it is possible to provide for a plurality of bearing surface positions with just one head part by altering its rotational position. This allows for micro adjustment with one head part, and also for a number of head parts to provide a greater number of possible results.

## Claims

1. A femoral prosthesis comprising a stem part and a head part, in which the stem part comprises a body section for location in the femur of a patient and a neck section comprising a spigot, in which the head part comprises a bearing surface formed as part of a sphere and a reverse side, in which the bearing surface comprises a polar axis extending through a centre of the sphere formed thereby, in which a bore is provided in said reverse side adapted to receive said spigot, in which said bore comprise a bore axis, and in which said bore axis is oblique to said polar axis.

2. A femoral prosthesis as claimed in claim 1 in which said bore axis intersects said polar axis.

3. A femoral prosthesis as claimed in claim 1 in which said bore axis is displaced from said polar axis at all points along the length of the bore axis.

4. A femoral prosthesis as claimed in any of claims 1 to 3 in which an outer surface of the spigot or an inner surface of the bore is provided with a protrusion, in which the other of said outer surface or said inner surface is provided with a co-operating indent, in which the protrusion is disposed in the indent when the head part is fitted onto the spigot, and in which at least part of the protrusion is radially distorted from the bore axis such that rotation of the head part about the bore axis is prevented.

5. A femoral prosthesis as claimed in claim 4 in which the protrusion is symmetrical about the bore axis.

6. A femoral prosthesis as claimed in claim 5 in which the bore comprises a base surface at an inner end thereof, in which the spigot comprises a free end face, and in which said protrusion is formed on said base surface and said indent on said free end face.

7. A femoral prosthesis as claimed in claim 6 in which said protrusion comprises a central portion and one or more arm portions extending therefrom, and in which said indent comprises a co-operating shape.

8. A femoral prosthesis as claimed in claim 7 in which said protrusion comprises four arm portions arranged at 90 degree intervals, and in which said indent comprises a co-operating shape.

9. A femoral prosthesis as claimed in claim 6 in which said protrusion comprises a geometric shape with a plurality of sides, and in which said indent comprises a co-operating shape.

10. A femoral prosthesis as claimed in claim 4 in which the bore comprises an annular and tapered bore side wall, in which the spigot comprises a co-operating annular and tapered spigot side wall, in which the protrusion is disposed on said bore side wall and said indent on said spigot side wall.

11. A femoral prosthesis as claimed in claim 10 in which the protrusion comprises one or more walls extending from said bore side wall towards said bore axis, and in which said indent comprises one or more co-operating troughs formed in the spigot side wall.

12. A femoral prosthesis as claimed in claim 4 in which the bore comprises a base surface at an inner end thereof, in which the spigot comprises a free end face, and in which said protrusion is formed on said free end face and said indent on said base surface.

13. A femoral prosthesis as claimed in any of claims 4 to 12 in which the head part comprises one or more position markings, in which the neck section comprises one or more complimentary position markings, and in which alignment of said position markings corresponds to said protrusion being aligned for disposition with, or being disposed in, said indent.

14. A stem part of a femoral prosthesis for use in a femoral prosthesis as claimed in any of claims 4 to 13.

15. A head part of a femoral prosthesis for use in a femoral prosthesis as claimed in any of claims 1 to 13.

16. A kit of parts comprising a femoral prosthesis as claimed in any of claims 1 to 13 and one or more trial heads, in which each of said one or more trial heads comprises a trial bearing surface formed as part of a sphere and a reverse side, in which the trail bearing surface comprises a polar axis extending through a centre of the sphere formed thereby, in which a bore is provided in said reverse side adapted to receive said spigot, in which said bore comprise a bore axis, and in which said bore axis is oblique to said polar axis.
